# EUROPEAN PATENT APPLICATION

(11) **EP 3 275 540 A1**
(43) Date of publication of application: **31.01.2018**
(21) Application number: 17183154.8
(22) Date of filing: 25.07.2017
(51) Int. Cl.: B01J 31/18, B01J 31/04, B01J 31/14, B01J 31/22, B01J 31/24, B01J 37/04, C07C 2/34

(54) **AN OLEFIN OLIGOMERIZATION, A CATALYST COMPOSITION THEREFOR AND A METHOD OF PREPARING THE CATALYST COMPOSITION**

(30) Priority: 26.07.2016 IN 201621025571
(71) Applicant: Reliance Industries Limited, Mumbai, Maharashtra 400021 (IN)
(72) Inventor: TEMBE, Gopal Laxman, 390012 Vadodara (IN); PILLAI, Muthukumaru Subramania, 391101 Vadodara (IN); MUKHERJEE, Soumen, 390021 Vadodara (IN); PAL, Nitin, 321001 Bharatpur (IN); JASRA, Raksh Vir, 390008 Vadodara (IN); VAKIL, Suketu M., 400025 Mumbai (IN)
(74) Representative: Linage González, Rafael

(57) **Abstract**

The present invention envisages a catalyst composition for olefin oligomerization. The composition comprises: (i) an aquated chromium salt, preferably Cr(III) 2-Et-hexanoate hydrate or Cr(III)acac hydrate; (ii) at least one organic ligand, preferably 2,5-dimethylpyrrole; (iii) a first co-catalyst, preferably trialkyl aluminum, especially Et3Al, and a second co-catalyst, preferably Et2AlCl; (iv) at least one fluid medium, preferably toluene, cyclohexane.

The present invention also provides the offline and inline methods for preparing the catalyst composition. The catalyst composition of the present invention provides high productivity and selectivity for 1-hexene during olefin oligomerization.

## Description

### FIELD

The present disclosure relates to an olefin oligomerization and the catalyst composition for olefin oligomerization.

### DEFINITIONS

As used in the present disclosure, the following terms are generally intended to have the meaning as set forth below, except to the extent that the context in which they are used indicate otherwise.

**Aquated compound:** A complex of a compound with water.

### BACKGROUND

The trimerization of ethylene to prepare 1-hexene, also produces by-products such as polyethylene, internal olefins and higher alpha olefin oligomers. Formation of the by-products reduces overall catalyst productivity for the preparation of 1-hexene and affects the economics of commercial production.

In the conventional processes of olefin trimerization, the catalyst preparation is carried out using a separate operation. The catalyst, when generated separately, necessitates isolation, storage and transportation to the polymerization reactor.

In order to efficiently use the separately generated catalyst, the catalyst needs to have a longer shelf life and should not decompose easily, when stored.

In an in-line method the generated catalyst can be directly transported without isolation or storage, to a polymerization reactor. Some of the possible benefits of the in-line method include process simplification and reduced capital and operating costs.

There is, therefore, felt a need for a method with an improved process and catalyst composition, which reduces the co-production of polyethylene (less than 0.2 wt %) and provides 1-hexene with high purity (> 99% alpha purity).

There also remains a need to improve the performance of trimerization from a catalyst composition generated through continuous contacting (in situ) of a metal precursor, ligand and activators to get the maximum selectivity and productivity for oligomers such as 1-hexene. Further, there is a need of method for generating catalyst from a chromium precursor that is inexpensive and easy.

### OBJECTS

Some of the objects of the present disclosure, which at least one embodiment herein satisfies, are as follows.

It is an object of the present disclosure to ameliorate one or more problems of the prior art or to at least provide a useful alternative.

An object of the present disclosure is to provide a catalyst composition for olefin oligomerization.

Still another object of the present disclosure is to provide a catalyst composition having high productivity for oligomerization of ethylene to 1-hexene.

Yet another object of the present disclosure is to provide a catalyst composition which can provide high selectivity for 1-hexene.

Other objects and advantages of the present disclosure will be more apparent from the following description, which is not intended to limit the scope of the present disclosure.

### SUMMARY

In accordance with one aspect of the present disclosure, there is provided a catalyst composition for olefin oligomerization, the catalyst composition comprises an aquated chromium salt; at least one organic ligand; a first co-catalyst and a second co-catalyst; at least one fluid medium; and optionally at least one electron donor.

In accordance with another aspect of the present disclosure, there is provided a method for preparing a catalyst composition; the method comprises i. mixing predetermined amounts of the aquated chromium salt, the organic ligand, the fluid medium and optionally, at least one electron donor selected from the group consisting of tris(4-methoxyphenyl) phosphine, 2,5-dimethylthiophene and cis-2,6-dimethylpiperidine to obtain a first mixture; ii. mixing predetermined amounts of at least one first co-catalyst, at least one second co-catalyst and the fluid medium to obtain a second mixture; and iii. admixing the first mixture and the second mixture to obtain a third mixture comprising the catalyst composition.

In accordance with the embodiments of the present disclosure, the aquated chromium salt is at least one selected from the group consisting of Cr(2-ethylhexanoate)₃.nH₂O, wherein integer 'n' is in the range of 1 to 6, and aquated chromium tris acetylacetonate.

The organic ligand is at least one selected from 2,5-Dimethylpyrrole, tris(4-methoxyphenyl) phosphine, 2,5-dimethylthiophene and cis-2,6-dimethylpiperidine.

The first co-catalyst is at least one selected from the group consisting of triethylaluminum, tridecylaluminum, tri-n-butylaluminum, tri-isopropylaluminum, tri-isoprenylaluminum, tri-isobutylaluminum, triphenylaluminum, tri-n-octylaluminum and tri-n-decylaluminum.

The second co-catalyst is at least one selected from the group consisting of ethyl aluminum sesquichloride, diethylaluminum chloride, di-isobutyl aluminum chloride and ethyl aluminum dichloride.

The fluid medium is at least one selected from the group consisting of toluene, hexane, cyclohexane, ethylbenzene, heptane and benzene.

The molar ratio of the aquated chromium salt to the organic ligand is in the range of 1:1 to 1:4. The molar ratio of the aquated chromium salt to the first co-catalyst is in the range of 1:5 to 1:15. The molar ratio of the first co-catalyst to the second co-catalyst is in the range of 1:2 to 1:7.

In accordance with still another aspect of the present disclosure, there is provided a method for olefin oligomerization using the catalyst composition; the method comprising the following steps: a. introducing to an oligomerization reactor, the third mixture comprising the catalyst composition, predetermined amount of ethylene and afluid medium under inert atmosphere to obtain a first slurry; b. oligomerizing the ethylene in the presence of the catalyst composition, under stirring at a predetermined speed, at a predetermined temperature, at a predetermined pressure and for a predetermined time period to obtain a second slurry comprising oligomer; c. adding a terminating agent to the second slurry; and d. separating and drying the oligomer.

In accordance with the embodiments of the present disclosure, the predetermined speed is in the range of 200 to 600 rpm; the predetermined temperature is in the range of 28 to 120 °C; the predetermined pressure is in the range of 5 to 50 kg/cm²; and the predetermined time period is in the range of 30 to 180 minutes.

In accordance with yet another aspect of the present disclosure, there is provided a method for olefin oligomerization using an in-line prepared catalyst composition, the method comprises the following steps: i. mixing predetermined amounts of an aquated chromium salt, an organic ligand, a fluid medium and optionally, at least one electron donor selected from the group consisting of tris(4-methoxyphenyl) phosphine, 2,5-dimethylthiophene and cis-2,6-dimethylpiperidine in a first reactor to obtain a first mixture; ii. mixing predetermined amounts of at least one first co-catalyst, at least one second co-catalyst and the fluid medium in a second reactor to obtain a second mixture; iii. allowing the second mixture to flow and contact the first mixture at a rate in the range of 2 mL/minute to 7 mL/minute to obtain a catalyst composition; iv. leading the catalyst composition to an oligomerization reactor; v. charging the oligomerization reactor with ethylene till a pressure 5 to 50 kg/cm² is attained; vi. heating the reactor at a temperature in the range of 30 to 120 °C and for a time period in the range of 30 to 180 minutes to obtain a slurry comprising oligomer; and vii. separating the oligomer from the slurry.

In accordance with the present disclosure, the method further comprises mixing the at least one electron donor selected from the group consisting of tris(4-methoxyphenyl) phosphine, 2,5-dimethylthiophene and cis-2,6-dimethylpiperidine, in step 'i'.

### DETAILED DESCRIPTION

In accordance with one aspect of the present disclosure, there is provided a catalyst composition for olefin oligomerization. The catalyst composition comprises an aquated chromium salt, at least one organic ligand, a first co-catalyst and a second co-catalyst, at least one fluid medium and optionally, at least one electron donor.

In accordance with the embodiments of the present disclosure, the aquated chromium salt is at least one selected from the group consisting of Cr(2-ethylhexanoate)₃.nH₂O, wherein integer 'n' is in the range of 1 to 6, and aquated chromium tris acetylacetonate.

In accordance with the embodiments of the present disclosure, the organic ligand is 2,5-Dimethylpyrrole, tris(4-methoxyphenyl) phosphine, 2,5-dimethylthiophene and cis-2,6-dimethylpiperidine.

In accordance with the embodiments of the present disclosure, the first co-catalyst is at least one selected from the group consisting of triethylaluminum, tridecylaluminum, tri-n-butylaluminum, tri-isopropylaluminum, tri-isoprenylaluminum, tri-isobutylaluminum, triphenylaluminum, tri-n-octylaluminum and tri-n-decylaluminum.

In accordance with the embodiments of the present disclosure, the second co-catalyst is at least one selected from the group consisting of ethyl aluminum sesquichloride, diethylaluminum chloride and di-isobutyl aluminum chloride and ethyl aluminum dichloride.

In accordance with the embodiments of the present disclosure, the fluid medium is at least one selected from the group consisting of toluene, hexane, cyclohexane, ethylbenzene, heptane and benzene.

In accordance with the embodiments of the present disclosure, the electron donor is at least one selected from the group consisting of tris(4-methoxyphenyl) phosphine, 2,5-dimethylthiophene and cis-2,6-dimethylpiperidine.

In accordance with the embodiments of the present disclosure, the molar ratio of the aquated chromium salt to the organic ligand is in the range of 1:1 to 1:4.

In accordance with the embodiments of the present disclosure, the molar ratio of the aquated chromium salt to the first co-catalyst is in the range of 1:5 to 1:15.

In accordance with the embodiments of the present disclosure, the molar ratio of the first co-catalyst, to the second co-catalyst is in the range of 1:2 to 1:7.

In accordance with the embodiments of the present disclosure, the catalyst composition can be stored at least for 180 days, prior to its use without affecting its catalytic activity.

In accordance with another aspect of the present disclosure, there is provided a method for preparing a catalyst composition for olefin oligomerization. The method comprises the steps, which are described herein below.

Initially, predetermined amounts of the aquated chromium salt, the at least one organic ligand, the fluid medium are mixed to obtain a first mixture. Similarly, predetermined amounts of the first co-catalyst, the second co-catalyst and the first fluid medium are mixed to obtain a second mixture. Finally, the first mixture and the second mixture are admixed to obtain the catalyst composition.

Optionally, in the method of the present disclosure, an electron donor can be added to the first mixture prior to admixing it with the second mixture.

Admixing the first mixture and the second mixture to produce the catalyst composition. The catalyst composition can be used for olefin oligomerization without isolation, separation and filtration.

In accordance with still another aspect of the present disclosure, there is provided a method for oligomerization. The method comprises the following steps:
At least one fluid medium, a predetermined amount of catalyst composition a predetermined amount of ethylene are introduced into an oligomerization reactor to obtain a reaction mixture.

Oligomerizing ethylene under stirring at a predetermined temperature and at a predetermined pressure for a predetermined time period to obtain a slurry comprising oligomer.

The oligomerization reaction is terminated by adding at least one terminating agent to the reactor followed by separating a solid mass from the slurry. The separation is achieved by known separation techniques such as filtration, evaporation, decantation, drying and heating under reduced pressure.

Optionally, the catalyst composition is aged before using it for oligomerization of ethylene.

In accordance with the embodiments of the present disclosure, the oligomerization is carried out at a temperature in the range of 28 °C to 120 °C.

In accordance with the embodiments of the present disclosure, the predetermined pressure is in the range of 5 to 50 kg/cm², typically 25 kg/cm².

In accordance with the embodiments of the present disclosure, the predetermined time period is in the range of 30 min to 180 min.

In accordance with the embodiments of the present disclosure, the step of stirring is carried out at a speed in the range of 200 to 600 rpm.

In accordance with one embodiment of the present disclosure, the oligomerization is terminated by adding 0.5% solution of di-tertbutyl-para-cresol in methanol to the first slurry.

The catalyst composition is used for olefin oligomerization, typically ethylene oligomerization. In accordance with the embodiments of the present disclosure, the productivity of the catalyst composition is in the range of 25 Kg/ g of Cr to 45.8 Kg/ g of Cr.

It is observed that the catalyst composition of the present disclosure provides selectivity for 1-hexene during ethylene oligomerization and produces less amounts of by-products.

Further, the catalyst composition prepared using the method of the present disclosure as discussed above can be stored in a flask and can be used as per requirement. The catalyst composition of the present disclosure has a shelf life of up to 5 months. The shelf life of the catalyst composition is determined by ageing the catalyst composition for varying time periods before using it for olefin oligomerization.

In accordance with yet another aspect of the present disclosure, there is provided a method for olefin oligomerization using an in-line prepared catalyst composition, the method comprises the following steps: Initially, predetermined amounts of the aquated chromium salt, at least one organic ligand, a fluid medium and optionally, at least one electron donor selected from the group consisting of tris(4-methoxyphenyl) phosphine, 2,5-dimethylthiophene and cis-2,6-dimethylpiperidine are mixed in a first reactor to obtain a first mixture.

Similarly, predetermined amounts of at least one first co-catalyst, at least one second co-catalyst and the fluid medium are mixed in a second reactor to obtain a second mixture.

Further, the first mixture and the second mixture are allowed to flow and contact each other at a rate in the range of 2 mL/minute to 7 mL/minute, so as to keep the contact time of the first mixture and the second mixture in the range of 1 minute to 30 minutes to obtain the catalyst composition.

The catalyst composition is then transferred to an oligomerization reactor. The oligomerization reactor is further charged with ethylene till a pressure in the range of 5 to 50 kg/cm² is attained.

Oligomerization of ethylene is performed by heating the oligomerization reactor at a temperature in the range of 28 to 120 °C and for a time period in the range of 30 to 180 minutes to obtain a third slurry comprising oligomer. The oligomer is separated from the third slurry.

The method of oligomerization using the in-line prepared catalyst composition, does not require the catalyst composition to be prepared separately, instead the oligomerization takes place in an in-line system wherein the first reactor, the second reactor and the oligomerization reactor are connected to each other through fluidic connections.

The method of the present disclosure uses commonly available and inexpensive reagents and fluid media and recycles the fluid media as well as some of the by-products. Hence, the method of the present disclosure is simple and economical.

The present disclosure is further described in light of the following experiments which are set forth for illustration purpose only and not to be construed for limiting the scope of the disclosure. The following experiments can be scaled up to industrial/commercial scale and the results obtained can be extrapolated to industrial scale.

### EXPERIMENTS

**The Experiment 1 (Catalyst Composition A):** 9.33 g aquated chromium (III) 2-ethylhexanoate was mixed with 3.11 mL (2.90g) 2,5-dimethylpyrrole in 75 mL toluene solution in a first vessel to obtain a first mixture. Similarly, 127 mL triethylaluminum (10 wt% in toluene) and 97 mL diethylaluminum chloride (10 wt% in toluene) were mixed in a second vessel to obtain a second mixture. The first mixture and the second mixture were admixed at 38 °C to obtain a catalyst composition **A.** The molar proportion of aquated chromium (III) 2-ethylhexanoate : DMP : TEAL: DEAC was 1: 3.5 : 10.5 : 8.

**Experiment 2 (Catalyst Composition B):** 1.27 g aquated chromium (III) 2-ethylhexanoate was mixed with 0.22 mL (0.20g) 2,5-dimethylpyrrole in 75 mL toluene solution in a first vessel to obtain a first mixture. Similarly, 16.7 mL triethylaluminum (10 wt% in toluene) and 12.8 mL diethylaluminum chloride (10 wt% in toluene) were mixed in a second vessel to obtain a second mixture. The first mixture and the second mixture were admixed at 38 °C to obtain a catalyst composition **B.** The molar proportion of aquated chromium (III) 2-ethylhexanoate : DMP : TEAL: DEAC was 1: 1.8 : 10.25 : 8.

**Experiment 3 (Catalyst Composition C):** 1.28 g aquated chromium (III) 2-ethylhexanoate was mixed with 0.26 mL (0.24g) 2,5-dimethylpyrrole in 10.5 mL toluene solution in a first vessel to obtain a first mixture. Similarly, 10.3 mL triethylaluminum (10 wt% in toluene) and 6.8 mL diethylaluminum chloride (10 wt% in toluene) were mixed in a second vessel to obtain a second mixture. The first mixture and the second mixture were admixed at 38 °C to obtain a catalyst composition **C.** The molar proportion of aquated chromium (III) 2-ethylhexanoate : DMP : TEAL: DEAC was 1: 2.1 : 6.3 : 4.2.

The catalyst compositions A, B and C were tested for 1-hexene selectivity, productivity and oligomer formation. The results are shown in Table 1.

### Experiment 4. Oligomerization of ethylene using the catalyst compositions A, B and C

2 litre Büchi reactor was charged with 800 mL of cyclohexane. The reactor was maintained at 50 °C. This was followed by the addition of 20 mL of catalyst composition **A** prepared in experiment 1 and the resultant mixture was aged for 24 hours. The catalyst composition and cyclohexane present in the reactor were thoroughly mixed by using a paddle type stirrer at a speed of 400 rpm. Ethylene gas was fed to the reactor till a pressure of 25 kg/cm² was attained, while the reactor temperature was increased to 90 °C. The oligomerization reaction was monitored by an exotherm and the exothermicity of the reaction was controlled by cooling the reactor by a circulating bath containing chilled water. Stirring was continued for 1 hour and, thereafter ethylene feed was disconnected. The reactor was cooled to 18 °C and degassed carefully by venting out excess ethylene. The contents of the reactor were quenched by injecting 5% acidic methanol, which is used as a terminating agent. The products were collected and weighed separately.

A total of 735 g liquid product was obtained along with some gaseous products. The composition of the gas and the liquid samples were analysed by Gas chromatography using a 30M (0.53mmID) capillary column Rtx-1 and flame ionization detector respectively. The weight of 1-hexene in the liquid phase was 679 g. 1-hexene selectivity was found to be greater than 99.1 % and the by-product i.e. polyethylene was 0.28 gm (0.04 wt %). The productivity of catalyst was 27.0 Kg/ gCr

### Experiments 5 to 15. Oligomerization of ethylene using catalyst compositions A, B and C

Experiments 5 to 12 were performed using similar procedure as that of experiment 4. Experiments were performed by changing various fluid medium used during oligomerization and varying ageing time. The results for the oligomerization reaction are provided in **Table 1** given below:

**Table 1. Oligomerization of ethylene using catalyst compositions A, B and C**

| **Experiment** | **Catalyst composition** | **Fluid medium** | **Ageing time** | **1-hexene selectivity (%)** | **Polyethylene recovered (%)** | **Productivity of the catalyst Kg/g of Cr** |
|---|---|---|---|---|---|---|
| **4** | A | cyclohexane | 24 hours | 99.1 | 0.04 | 27 |
| **5** | A | cyclohexane | 60 days | 99.4 | 0.09 | 20 |
| **6** | A | cyclohexane | 90 days | 99.1 | 0.06 | 22.6 |
| **7** | A | cyclohexane | 120 days | 99.4 | 0.03 | 23.5 |
| **8** | A | cyclohexane | 150 days | 99.2 | 0.09 | 17.5 |
| **9** | A | Cyclohexane (presaturated with ethylene pressure of 8 kg/cm²) | 5 days | 99.0 | 0.15 | 32.6 |
| **10** | A | Cyclohexane + 1-hexene (1:1) | 45 days | 98.9 | 3.66 | 11.4 |
| **11** | A | Cyclohexane (presaturated with ethylene pressure of 8 kg/cm²) | 15 days | 99.0 | 3.19 | 17.2 |
| **12** | A | 459 mL Cyclohexane + 360 mL of 1-hexene | 30 days | 99.3 | 0.19 | 15 |
| **13** | A | cyclohexane | 45 days | 99.3 | 0.09 | 14 |
| **14** | B | cyclohexane | 24 hours | 99.2 | 0.1 | 21.1 |
| **15** | C | cyclohexane | 15 days | 99.0 | 0.71 | 4.5 |

From **Table 1,** it is evident that the productivity of the catalyst composition A is in the range of 11 to 32.6 Kg 1-hexene per gram of chromium. On ageing the catalyst composition, the activity was found to deteriorate after 120 days (experiments 6, 7 and 8). The selectivity for 1-hexene prepared using the catalyst composition **A** of the present disclosure is found to be greater than 98%. Cyclohexane is found to be the preferred fluid medium for carrying out the oligomerization of ethylene.

### Experiment 16: Olefin oligomerization using the in-line prepared catalyst composition

### (Catalyst Composition D)

A first vessel was charged with 0.8 g aquated chromium (III) 2-ethylhexanoate and 0.38 g 2,5-dimethylpyrrole dissolved in 10 mL toluene to obtain a first mixture. Similarly, 16.7 mL of triethylaluminum (10 wt% in toluene) and 12.8 mL diethylaluminum chloride (10 wt% in toluene) were mixed in a second vessel to obtain a second mixture. The first mixture and the second mixture were allowed to flow continuously at a flow rate of 2.5 mL/min, through a coiled reaction tube having 5 mm diameter and length of 2 feet cooled to 20 °C. The contact time of the first mixture and the second mixture was so adjusted that an instantaneous reaction occurred to generate an active catalyst composition inside the coiled reaction tube. The reduction of chromium and the accompanying colour change indicated the formation the active catalyst composition. The first mixture and the second mixture on contacting with each other formed a third mixture. The third mixture was aged for 24 hours.

The third mixture on ageing was transferred to a 2 litre Büchi reactor containing 800 mL of cyclohexane at 50 °C. The contents of the reactor were thoroughly mixed by using a paddle type stirrer at a speed of 400 rpm. Ethylene was fed to the reactor to attain a pressure of 25 kg/cm², while the reactor temperature was maintained at 90 °C. Stirring was continued for 1 hour and the reaction was stopped by disconnecting the ethylene feed. The liquid content of the reactor was quenched by injecting 5% acidic methanol. Products were separated from the reactor.

A total of 437 g liquid product was obtained along with some gaseous products. The composition of the gas and the liquid samples were analysed by gas chromatography using a 30M (0.53mmID) capillary column Rtx-1 and flame ionization detector respectively. The combined weight of 1-hexene and in the liquid phase was 407 g. 1-hexene selectivity was found to be 99.4 % and solid polyethylene recovered from the reactor corresponded to 0.12 gm (0.03 wt %). The productivity of the catalyst was 17.1 Kg/ g of Cr.

**Experiment 17 (Catalyst composition E) :** A first vessel was charged with 341 g aquated chromium (III) 2-ethylhexanoate and 107 g 2,5-dimethylpyrrole dissolved in 2.5 L toluene to obtain a first mixture. Similarly, 4.8 L of triethylaluminum (10 wt% in toluene) and 3 L diethylaluminum chloride (10 wt% in toluene) were mixed in a second vessel to obtain a second mixture. The first mixture and the second mixture were allowed to flow continuously at a flow rate of 0.1 kg/min to about 5 mL/min through a coiled reaction tube having 0.25 inch in diameter and length of 10 feet that was cooled to 20 °C. The contact time of the first mixture and the second mixture was so adjusted that an instantaneous reaction occurred to generate a catalyst composition **E** inside the coiled reaction tube. The reduction of chromium and the accompanying colour change indicated the formation of the active catalyst composition **E.** The first mixture and the second mixture on contacting with each other formed a third mixture, which was directly employed for ethylene oligomerization.

### Use of electron donor for preparing the catalyst compositions

**Experiment 18 (Catalyst composition F):** Aquated chromium (III) 2-ethylhexanoate (2.38 g) and 2,5-dimethylpyrrole (0.66 g) were dissolved in 15.0 mL toluene in a Schlenk flask. To this was added tris(4-methoxyphenyl) phosphine (0.19 g) in 5.0 mL toluene. The contents were stirred for 2 hours. A mixture of triethylaluminum (29.0 mL, 10wt% in toluene) and diethylaluminum chloride (24.0 mL, 10wt% in toluene) were added to the reactor under a nitrogen purge at 28 °C to obtain a reaction mixture containing a catalyst composition. The temperature of the reaction mixture was increased by 3 °C during catalyst preparation. The contents of the reactor stood overnight and were used as formed. The overall molar ratio of chromium precursor: DMP: TMP: TEAL: DEAC was 1: 3.5: 0.25: 11: 9.

**Experiment 19 (Catalyst composition G):** Aquated chromium (III) 2-ethylhexanoate (2.38 g) and 2,5-dimethylpyrrole (0.50 g) were dissolved in 15.0 mL toluene in a Schlenk flask. 2,5-Dimethylthiophene (0.07 g) in 5.0 mL toluene was added to the Schlenk flask. The contents were stirred for 2 hours. A mixture of triethylaluminum (30.0 mL, 10wt% in toluene) and diethylaluminum chloride (25.0 mL, 10wt% in toluene) were added under a nitrogen purge at 28 °C to the Schlenk flask to obtain a reaction mixture containing the catalyst composition. The temperature of the reaction mixture increased by about 2-3 °C during catalyst preparation. The contents of the reactor stood overnight and were used as formed. The overall molar ratio of chromium precursor: DMP: DMT: TEAL: DEAC was 1: 2.5: 0.3: 11: 9.

**Experiment 20 (Catalyst composition H) :** Aquated chromium (III) 2-ethylhexanoate (2.40 g) and 2,5-dimethylpyrrole (0.52 g) were dissolved in 15.0 mL toluene in a Schlenk flask. *cis*-2,6-Dimethylpiperidine (0.06 g) in 5.0 mL toluene was added to the Schlenk flask. The contents were stirred for 2 hours. A mixture of triethylaluminum (30.0 mL, 10wt% in toluene) and diethylaluminum chloride (25.0 mL, 10wt% in toluene) were then added to the reactor under a nitrogen purge at 28 °C to obtain a reaction mixture containing a catalyst composition. The temperature of the reaction mixture increased by about 2-3 °C during catalyst preparation. The contents of the reactor stood overnight and were used as formed. The overall molar ratio of Chromium precursor: DMP: PIP: TEAL: DEAC was 1: 2.5: 0.3: 11: 8.

**Table 2:**

| **Experiment** | **Catalyst composition** | **Solvent** | **Ageing time** | **1-hexene selectivity (%)** | **Polyethylene recovered (%)** | **Productivity of the catalyst Kg/g of Cr** |
|---|---|---|---|---|---|---|
| **16** | D | cyclohexane | 1day | 99.4 | 0.03 | 17.1 |
| **17** | E | cyclohexane | 24 hours | 99.1 | 0.01 | 39.5 |
| **18** | F | cyclohexane | 24 hours | 99.0 | 0.03 | 6.7 |
| **19** | G | cyclohexane | 5 days | 99.2 | 0.04 | 12.7 |
| **20** | H | cyclohexane | 90 days | 99.3 | 0.04 | 18.1 |

From above table it is clear that the catalyst compositions D, E, F, G and H provided productivity in the range of 6.7 to 39.5 Kg/g of Cr. The selectivity for 1-hexene is found to be more than 99%. The formation of polyethylene, which is a by-product is less than 0.04%

Further, the catalyst compositions of the present disclosure are found to be helpful in achieving more than 99% selectivity for 1-hexene during ethylene oligomerization. The by-product is formed in lesser amounts.

**The following experiments represent scaling of the process of the present disclosure to industrial/commercial scale.**

### Experiment 21: Oligomerization of ethylene using the catalyst composition B

20 litre Büchi reactor was charged with 6728 mL of cyclohexane. The reactor was maintained at 50 °C. This was followed by the addition of 173 mL of catalyst composition **B** prepared in experiment 2 and the resultant mixture was aged for 24 hours. The catalyst composition and cyclohexane present in the reactor were thoroughly mixed by using a paddle type stirrer at a speed of 400 rpm. Ethylene gas was fed to the reactor till a pressure of 27 kg/cm² was attained, while the reactor temperature was increased to 90 °C. The oligomerization reaction was monitored by an exotherm and the exothermicity of the reaction was controlled by cooling the reactor by a circulating bath containing chilled water. Stirring was continued for 1 hour and, thereafter ethylene feed was disconnected. The reactor was cooled to 18 °C and degassed carefully by venting out excess ethylene. The contents of the reactor were quenched by injecting 104 mL of 1-octanol, which is used as a terminating agent. The products were collected and weighed separately.

A total of 5111 g liquid product was obtained along with some gaseous products. The composition of the gas and the liquid samples were analysed by Gas chromatography using a 30M (0.53mmID) capillary column Rtx-1 and flame ionization detector respectively. The weight of 1-hexene in the liquid phase was 5022 g. 1-hexene selectivity was found to be greater than 99.0 % and the by-product i.e. polyethylene was 10.5 g (0.04 wt %). The productivity of catalyst was 19.0 Kg/ gCr

| **Experiment** | **Catalyst composition** | **Fluid medium** | **Ageing time** | **1-hexene selectivity (%)** | **Polyethylene recovered (%)** | **Productivity of the catalyst Kg/g of Cr** |
|---|---|---|---|---|---|---|
| **21** | B | cyclohexane | 24 hours | 99.0 % | 0.04 wt % | 19.0 Kg/ gCr |

### Experiment 22: Olefin oligomerization using the in-line prepared catalyst composition (Catalyst Composition E)

A first vessel was charged with 178.53 g aquated chromium (III) 2-ethylhexanoate and 123.32 g 2,5-dimethylpyrrole dissolved in 500 mL toluene to obtain a first mixture. Similarly, 8.4 kg of triethylaluminum in toluene (10 wt% in toluene) and 6.7 Kg diethylaluminum chloride in toluene (10 wt% in toluene) were mixed in a second vessel to obtain a second mixture. The first mixture and the second mixture were allowed to flow continuously at a flow rate of 0.14kg/hr, through a coiled reaction tube having 0.25 inch diameter and length of 15 feet. The contact time of the first mixture and the second mixture was so adjusted that an instantaneous reaction occurred to generate an active catalyst composition inside the coiled reaction tube. The reduction of chromium and the accompanying colour change indicated the formation the active catalyst composition. The first mixture and the second mixture on contacting with each other formed a catalyst composition. The catalyst composition was aged for 24 hours.

The aged catalyst composition thus generated was continuously injected into the 90 liter Büchi reactor. The solvent cyclohexane for the reaction was injected at a flow rate of 10.4 kg/hr. The contents of the reactor were thoroughly mixed by using a paddle type stirrer at a speed of 400 rpm. Ethylene was fed to the reactor to attain a pressure of 25 kg/cm², while the reactor temperature was maintained at 90 °C. Stirring was continued for 24 hours and the reaction was stopped by disconnecting the ethylene feed. The liquid content of the reactor was quenched by injecting 5% acidic methanol.

Products were separated from the reactor. The liquid product containing hexene, solvent and higher oligomers were discharged continuously. After the first 24 hours, the first mixture was diluted by adding 1.8 Kg of toluene. The flow rates of first mixture and second mixture were maintained at 0.086 Kg/hour and 0.11 Kg/hour respectively for 19 hours. The molar ratio of the four catalyst components was Cr : DMP : TEAL: DEAC 1 : 3.5 : 15.4 : 11.6.

After 43hours the concentration of the first mixture was changed by diluting with 0.68 Kg of toluene. The flow rates of first mixture and second mixture were maintained at 0.057 Kg/hr for the next 12 hrs. The molar ratio of the four catalyst components was Cr : DMP : TEAL: DEAC 1 : 3.5 : 14.0 : 10.6.

At the end of a total continuous oligomerization run time of 55 hours the reaction was terminated by adding 1 kg of octanol to quench the active catalyst. The ethylene consumed as per MFT was 405.1 Kg and the total liquid product formed including trace quantity of polymer (0.05 Kg, 0.013%) was 378.8 Kg. Overall ethylene conversion was 93.5 %. The total 1-hexene content in the product after 55 hours of continuous oligomerization was 89 % with selectivity of 99.1 % as per GC analysis. The combined productivity was 39.5 Kg /g Cr.

The concentrations of the catalyst composition could be suitably adjusted at any time during the run to improve the overall yield of products as well as to minimize the formation of by-products.

The ageing studies proved that the catalyst compositions of the present disclosure can be stored up to 5 months and used as per convenience.

Further, the catalyst composition for olefin oligomerization can be prepared by the in-line method and used as it is without isolation, filtration or extraction of the catalyst.

### TECHNICAL ADVANCEMENTS

The present disclosure described herein above has several technical advantages including, but not limited to, the realization of a catalyst composition and a method for preparation thereof, that
- uses an aquated chromium salt as a precursor that is inexpensive,
- has high selectivity and high productivity for 1-hexene,
- reduces co-production of polyethylene, which is undesired product and
- is simple, efficient and industrially economical.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The use of the expression "at least" or "at least one" suggests the use of one or more elements or ingredients or quantities, as the use may be in the embodiment of the invention to achieve one or more of the desired objects or results. While certain embodiments of the inventions have been described, these embodiments have been presented by way of example only and are not intended to limit the scope of the inventions. Variations or modifications to the formulation of this invention, within the scope of the invention, may occur to those skilled in the art upon reviewing the disclosure herein. Such variations or modifications are well within the spirit of this invention.

The numerical values given for various physical parameters, dimensions and quantities are only approximate values and it is envisaged that the values higher than the numerical value assigned to the physical parameters, dimensions and quantities fall within the scope of the invention unless there is a statement in the specification to the contrary.

While considerable emphasis has been placed herein on the specific features of the preferred embodiment, it will be appreciated that many additional features can be added and that many changes can be made in the preferred embodiment without departing from the principles of the disclosure. These and other changes in the preferred embodiment of the disclosure will be apparent to those skilled in the art from the disclosure herein, whereby it is to be distinctly understood that the foregoing descriptive matter is to be interpreted merely as illustrative of the disclosure and not as a limitation.

## Claims

1. A catalyst composition for olefin oligomerization, said catalyst composition comprising:
i. an aquated chromium salt;
ii. at least one organic ligand;
iii. a first co-catalyst and a second co-catalyst;
iv. at least one fluid medium; and
v. optionally, at least one electron donor selected from the group consisting of tris(4-methoxyphenyl) phosphine, 2,5-dimethylthiophene and cis-2,6-dimethylpiperidine.

2. The catalyst composition as claimed in claim 1, wherein said aquated chromium salt is at least one selected from the group consisting of Cr(2-ethylhexanoate)₃.nH₂O, wherein said integer 'n' is in the range of 1 to 6 and aquated chromium tris acetylacetonate.

3. The catalyst composition as claimed in claim 1, wherein said organic ligand is 2,5-dimethylpyrrole, tris(4-methoxyphenyl) phosphine, 2,5-dimethylthiophene and cis-2,6-dimethylpiperidine.

4. The catalyst composition as claimed in claim 1, wherein said first co-catalyst is at least one selected from the group consisting of triethylaluminum, tridecylaluminum, tri-n-butylaluminum, tri-isopropylaluminum, tri-isoprenylaluminum, tri-isobutylaluminum, triphenylaluminum, tri-n-octylaluminum and tri-n-decylaluminum.

5. The catalyst composition as claimed in claim 1, wherein said second co-catalyst is at least one selected from the group consisting of ethyl aluminum sesquichloride, diethylaluminum chloride, di-isobutyl aluminum chloride and ethyl aluminum dichloride.

6. The catalyst composition as claimed in claim 1, wherein said fluid medium is at least one selected from the group consisting of toluene, hexane, cyclohexane, ethylbenzene, heptane and benzene.

7. The catalyst composition as claimed in claim 1, wherein the molar ratio of said aquated chromium salt to said organic ligand is in the range of 1:1 to 1:4.

8. The catalyst composition as claimed in claim 1, wherein the molar ratio of said aquated chromium salt to said first co-catalyst is in the range of 1:5 to 1:15.

9. The catalyst composition as claimed in claim 1, wherein the molar ratio of said first co-catalyst to said second co-catalyst is in the range of 1:2 to 1:7.

10. The catalyst composition as claimed in claim 1, wherein said catalyst composition can be stored up to 180 days under inert atmosphere, prior to its use.

11. A method for preparing a catalyst composition; said method comprising the following steps:
i. mixing predetermined amounts of an aquated chromium salt, an organic ligand, a portion of a fluid medium and optionally, at least one electron donor selected from the group consisting of tris(4-methoxyphenyl) phosphine, 2,5-dimethylthiophene and cis-2,6-dimethylpiperidine, to obtain a first mixture;
ii. mixing predetermined amounts of at least one first co-catalyst, at least one second co-catalyst and a portion of said fluid medium to obtain a second mixture; and
iii. admixing said first mixture and said second mixture under inert atmosphere to obtain said catalyst composition.

12. A method for olefin oligomerization using a catalyst composition, said method comprising the following steps:
a. introducing to an oligomerization reactor, a catalyst composition comprising an aquated chromium salt, at least one organic ligand, a first co-catalyst, a second co-catalyst, at least one fluid medium, and olefin under inert atmosphere to a pressure in the range of 5 to 50 kg/cm² to obtain a reaction mixture;
b. oligomerizing said olefin in the presence of said catalyst composition, while stirring said reaction mixture at a stirring speed in the range of 200 to 600 rpm, at a temperature in the range of 28 to 120 °C, and for a time period in the range of 30 to 180 minutes to obtain a slurry comprising an oligomer;
c. terminating the oligomerization by adding a terminating agent to said second slurry; and
d. separating and drying said oligomer.

13. The method as claimed in claim 12, wherein said terminating agent is at least one selected from the group consisting of methanol, ethanol and hexanol and octanol.

14. The method as claimed in claim 12, wherein said olefin is ethylene and said oligomer is 1-hexene.

15. The method as claimed in claim 12, wherein said catalyst composition is prepared inline by
i. mixing predetermined amounts of an aquated chromium salt, an organic ligand, a fluid medium and optionally, at least one electron donor selected from the group consisting of tris(4-methoxyphenyl) phosphine, 2,5-dimethylthiophene and cis-2,6-dimethylpiperidine in a first reactor to obtain a first mixture;
ii. mixing predetermined amounts of at least one first co-catalyst, at least one second co-catalyst and said fluid medium in a second reactor to obtain a second mixture;
iii. allowing said second mixture to flow and contact said first mixture at a rate in the range of 2 mL/minute to 7 mL/minute to obtain a catalyst composition; and
iv. leading said catalyst composition to an oligomerization reactor to carry out the oligomerization reaction.
